# EUROPEAN PATENT APPLICATION

(11) **EP 4 681 765 A1**
(43) Date of publication of application: **21.01.2026**
(21) Application number: 23927415.2
(22) Date of filing: 14.03.2023
(51) Int. Cl.: A61M 25/09

(54) **MEDICAL DEVICE**

(71) Applicant: ASAHI INTECC CO., LTD., Seto-shi, Aichi, 489-0071 (JP); Mustapha, Jihad A., Grandville, Michigan 49468 (US)
(72) Inventor: NISHIGISHI Makoto, Seto-shi, Aichi 489-0071 (JP)
(74) Representative: Winter, Brandl - Partnerschaft mbB
(86) International application number: PCT/JP2023/009924
(87) International publication number: WO 2024/189794

(57) **Abstract**

A medical device has a long main body and a protrusion protruding to the farther distal end side beyond the distal end of the main body. In the protrusion, the maximum value of the width of a contour when viewed from a 1st direction orthogonal to the central axis of the main body is larger than the maximum value of the width of a contour when viewed from a 2nd direction orthogonal to the central axis. In the 1st directional vision, the contour has a curved portion, and the width of the contour at the 1st central axis position is larger than the width of the contour at the 2nd central axis position located on the proximal end side of the 1st central axis position.

## Description

### Field

The technology disclosed herein relates to a medical device.

### Background

Methods using catheters are widespread as methods for treating or testing intravascular constricted parts or occluded parts (hereinafter referred to as "lesion(s)"). A guide wire is used for guiding a catheter to the position of an intravascular lesion. Guide wires are required to have high penetration performance in order to penetrate a relatively hard lesion such as chronic total occlusion.

Technology for processing the shape of the most distal end portion of a guide wire into an open lemon shape, a hook shape, a planar paddle shape or the like is known (for example, see Patent Literature 1 to Patent Literature 5).

### Citation List

### Patent Literature

[Patent Literature 1] Japanese Unexamined Patent Publication No. 2019-209152.
[Patent Literature 2] U.S. Unexamined Patent Application Publication No. 2018/0280051
[Patent Literature 3] U.S. Unexamined Patent Application Publication No. 2018/0280052
[Patent Literature 4] International Publication No. WO 2018/184017
[Patent Literature 5] U.S. Unexamined Patent Application Publication No. 2021/0121199

### Summary

### Technical Problem

The above-described conventional technology may improve the penetration performance of the guide wire. However, conventional guide wires still have a room for improvement in penetration performance.

Technology capable of solving the above problems is disclosed herein.

### Solution to Problem

The technology disclosed herein can be implemented as the following aspects, for example.

(1) A medical device disclosed herein includes a long main body and a protrusion that protrudes to the farther distal end side beyond the distal end of the main body, in which the maximum value of the width of a contour of the protrusion when viewed from a 1st direction orthogonal to the central axis of the main body is larger than the maximum value of the width of a contour of the protrusion when viewed from a 2nddirection orthogonal to the central axis. In the 1st directional vision, the contour includes a curved portion, and the width of the contour at a 1st central axis position is larger than the width of the contour at a 2nd central axis position located on the proximal end side of the 1st central axis position.

The protrusion of the medical device has, in the 1st directional vision, a shape in which the contour has a curved portion. Moreover, in the protrusion, the maximum value of the width of the contour in the 1st directional vision is larger than the maximum value of the width of the contour when viewed from the 2nd direction orthogonal to the central axis. Specifically, the protrusion has a non-rotationally symmetric shape. Accordingly, with the protrusion located in a lesion, the medical device is rotated around the central axis to rotate the protrusion around the central axis, so that the lesion can be efficiently perforated by the curved portion of the contour of the protrusion.

(2) The medical device may be configured so that the 1st direction is orthogonal to the 2nd direction. According to the medical device, the protrusion can be flat-shaped. Therefore, according to the medical device, resistance that occurs when the protrusion is manipulated to enter a lesion and when the protrusion is advanced to the distal side in the lesion can be reduced. As a result, the penetration performance of the guide wire can be effectively improved.

(3) The medical device may be configured so that the maximum value of the width of the contour in the 1st directional vision is the same as the maximum width of the protrusion. In the medical device, a direction in which the contour of the protrusion has a curved portion is consistent with a direction in which the protrusion has the maximum width. Therefore, according to the medical device, the width of a portion corresponding to the curved portion in the protrusion is maximized, so as to be able to effectively improve the performance of perforating a lesion by the rotation of the protrusion. As a result, the penetration performance of the medical device can be even more effectively improved.

(4) The medical device may be configured so that the surface of the protrusion has an edge that is a boundary of two surfaces. According to the medical device, the protrusion is rotated around the central axis, making it possible to perforate a lesion in such a manner that the lesion is cut up by the edge on the surface of the protrusion. As a result, the penetration performance of the medical device can be even more effectively improved.

(5) The medical device may be configured so that the surface of the protrusion has the edge on the contour in the 1st directional vision. According to the medical device, the edge can be arranged at the outermost peripheral position of the rotational track when the protrusion is rotated around the central axis, so that the edge of the rotating protrusion can be surely brought into contact with a lesion, and the performance of perforating the lesion by the rotation of the protrusion can be effectively improved. As a result, the penetration performance of the medical device can be extremely effectively improved.

(6) The medical device may be configured so that in the 1st directional vision, the width of the contour at the 1st central axis position of the protrusion is larger than the width of the contour at a 3rd central axis position located on the distal end side of the 1st central axis position. According to the medical device, the protrusion has a portion the diameter of which increases and then decreases from the proximal end side to the distal end side, so that the portion on the distal end side of the protrusion can be shaped to be highly capable of entering a lesion, as well as the width of a portion between the proximal end and the distal end in the protrusion is relatively increased so as to be able to improve the performance of perforating a lesion by the rotation of the protrusion. As a result, the penetration performance of the medical device can be further effectively improved.

(7) The medical device may be configured so that the main body includes a core wire configured of a metal wire and a coil body having a configuration in which one or more wires are wound around the outer periphery of the core wire, and being joined to the core wire. According to the medical device, the main body includes the core wire and the coil body. Hence, when the medical device is rotated around the central axis, the coil body is also rotated around the central axis, the screw-in action of the coil body having a spiral outer peripheral surface can ensure the advancement of the protrusion to the distal side in a lesion, and the penetration performance of the medical device can be even more effectively improved. According to the medical device, the torquability of the distal end portion of the medical device can be improved because of the presence of the coil body, as well as the flexibility of the distal end portion of the medical device can be improved. Moreover, in the unlikely event of the failure of the protrusion, the protrusion can be prevented from remaining in the body cavity.

(8) The medical device may have a configuration wherein the protrusion includes a loop part. According to the medical device, the protrusion can be shaped to have a portion with a relatively large width, and, a relatively thin linear portion, so that a lesion can be effectively perforated by the rotating protrusion and the penetration performance of the medical device can be extremely effectively improved.

(9) The medical device may be configured so that the protrusion has a loop part, the metal wire configuring the core wire has a distal end portion, a proximal end portion, and an intermediate portion located between the distal end portion and the proximal end portion, the distal end portion and the proximal end portion of the metal wire are joined with each other at a position covered by the coil body, and the intermediate portion of the metal wire configures the loop part. According to the medical device, a core wire having a loop-shaped protrusion can be formed using a single metal wire. As a result, compared to a configuration in which a protrusion is formed as a separate body, the protrusion can be prevented from leaving.

(10) The medical device may be configured so that the proximal end portion of the metal wire configuring the core wire has a 1st portion and a 2nd portion that is adjacent to the distal end side with respect to the 1st portion, is joined to the distal end portion of the metal wire, and has a transverse section having an area smaller than that of the transverse section of the 1st portion. The medical device can prevent a situation in the core wire such that the width of a portion configured by joining the 2nd portion of the proximal end portion of the metal wire and the distal end portion of the metal wire is excessively increased, resulting in an excessively increased rigidity gap between this portion and a portion configured of the 1st portion of the proximal end portion of the metal wire. Therefore, according to the medical device, the rigidity gap of the core wire can be reduced and the durability and the manoeuvrability of the medical device can be improved.

(11) The medical device may be configured so that the 2nd portion of the proximal end portion of the metal wire configuring the core wire has a tapered shape in which the width gradually decreases from the boundary position between the 2nd portion and the 1st portion to the distal end side. According to the medical device, compared to a configuration in which steps are made in the boundary position between the 2nd portion and the 1st portion of the proximal end portion of the metal wire, the rigidity gap of the metal wire itself configuring the core wire can be prevented from excessively increasing. As a result, the durability and the manoeuvrability of the medical device can be effectively improved.

(12) The medical device may be configured so that the coil body is configured of a multi-thread coil formed by winding a plurality of the wires around the outer periphery of the core wire. According to the medical device, the torquability and the flexibility of the distal end portion of the medical device can be effectively improved.

(13) The medical device may be configured so that the 1st central axis position may be located on the distal end side of the center of the central axis position of the protrusion. According to the medical device, penetration performance can be effectively improved.

Technology disclosed herein can be achieved in various forms, such as medical devices and methods for producing the same.

### Brief Description of Drawings

FIG. 1 is a diagram that schematically shows the configuration of a guide wire in a first embodiment.
FIG. 2 is a diagram that shows the detailed configuration of a core wire configuring the guide wire in the first embodiment.
FIG. 3 is a diagram that shows the detailed configuration of the core wire configuring the guide wire in the first embodiment.
FIG. 4 is a diagram that shows an example of a method for producing the core wire in the first embodiment.
FIG. 5 is a diagram that shows an example of a method for using the guide wire in the first embodiment.
FIG. 6 is a diagram that shows the detailed configuration of the core wire configuring a guide wire of the second embodiment.
FIG. 7 is a diagram that shows an example of a method for producing the core wire configuring the guide wire of the second embodiment.
FIG. 8 is a diagram that shows the detailed configuration of the core wire configuring a guide wire of a third embodiment.
FIG. 9 is a diagram that shows the detailed configuration of the core wire configuring a guide wire of a fourth embodiment.
FIG. 10 is a diagram that shows an example of a method for producing the core wire configuring the guide wire of the fourth embodiment.
FIG. 11 is a diagram that shows the detailed configuration of the core wire configuring a guide wire in a fifth embodiment.
FIG. 12 is a diagram that shows the detailed configuration of the core wire configuring a guide wire in a sixth embodiment.

### Description of Embodiments

### A. First embodiment:

### A-1. Configuration of guide wire 100

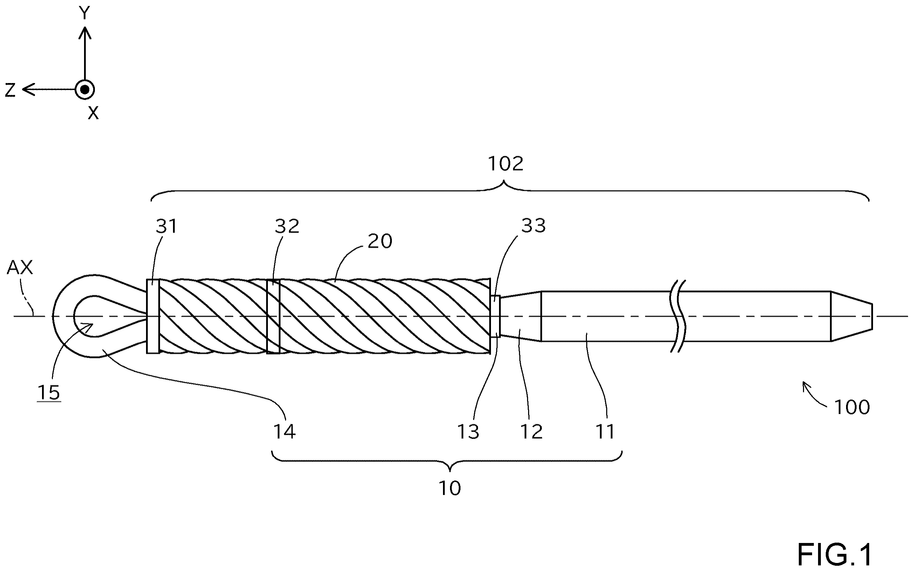

FIG. 1 is a diagram that schematically shows the configuration of the guide wire 100 in the first embodiment. FIG. 1 shows the configuration of a side (the side viewed from X-axis direction) of the guide wire 100. In FIG 1, Z axis positive direction side is the distal end side (distal side) to be inserted to a body, and Z axis negative direction side is the proximal end side (proximal side, near side) to be manipulated by a professional such as a doctor. In FIG. 1, a portion of the guide wire 100 is omitted. FIG. 1 shows a state where a central axis AX of the guide wire 100 is a straight line parallel to the Z axis direction. The guide wire 100 is flexible enough to be bent. The same applies to the following figures.

In the Description, regarding the guide wire 100 and each constituent member thereof, an end on the distal end side is referred to as "distal end", the distal end and a portion in the vicinity thereof are referred to as "distal end portion", an end on the proximal end side is referred to as "proximal end", and the proximal end and a portion in the vicinity thereof are referred to as "proximal end portion". The outer diameters of the guide wire 100 and that of each constituent member thereof each refers to the size or the width thereof along the direction orthogonal to the central axis AX. The longitudinal sections of the guide wire 100 and that of each constituent member thereof each refers to a cross section including the central axis AX of the guide wire 100, and the transverse sections of the guide wire 100 and that of each constituent member thereof each refers to a cross section orthogonal to the central axis AX.

The guide wire 100 is a long medical device that is inserted into a blood vessel in order to treat an intravascular lesion (constricted part or occluded part). The full length of the guide wire 100 ranges from about 1500 mm to 2000 mm, for example. The guide wire 100 includes the core wire 10 and a coil body 20.

The core wire 10 is a long member that extends along the central axis AX of the guide wire 100 and is configured of a metal wire. The core wire 10 has a thick diameter part 11, a thin diameter part 13 being located on the distal end side with respect to the thick diameter part 11 and having the diameter smaller than that of the thick diameter part 11, a tapered portion 12 being located between the thick diameter part 11 and the thin diameter part 13 and having the diameter that gradually decreases from the boundary position thereof with the thick diameter part 11 to the boundary position thereof with the thin diameter part 13, and a protrusion 14 located on the distal end side with respect to the thin diameter part 13. The shape of the transverse section (cross section XY) at each position (excluding the protrusion 14) of the core wire 10 can have any shape. For example, the shape of the transverse section at each position of the core wire 10 is circular or rectangular. The outer diameter of the thick diameter part 11 ranges from about 0.2 mm to 0.8 mm, for example. The configuration of the core wire 10 will be described in more detail as follows.

Hereinafter, a portion other than the protrusion 14 of the core wire 10 in the guide wire 100 is also referred to as a main body 102. The guide wire 100 has the long main body 102 and the protrusion 14 protruding to the farther distal end side beyond the distal end of the main body 102. Most parts of the main body 102 have circular cross sections along the entire length, and thus the outer diameter can be measured at each axis directional position.

Examples of materials to be used for forming the core wire 10 include stainless steel (e.g., SUS302, SUS304 and SUS316), an Ni-Ti alloy, and a piano wire. The core wire 10 may be entirely formed of the same material or materials that differ from one portion to another.

The coil body 20 is a hollow cylindrical coiled member formed by winding one or more wires around the outer periphery of the core wire 10. Each wire configuring the coil body 20 may be configured of a single strand or a twisted wire made of a plurality of single strands. In this embodiment, the coil body 20 is configured of a multi-thread coil formed by winding a plurality of wires and each wire configuring the coil body 20 is a twisted wire. In the embodiment, the coil body 20 covers substantially the entire thin diameter part 13 in the core wire 10. The outer diameter of the coil body 20 ranges from about 0.3 mm to 1.0 mm, for example. The coil body 20 may have a shape in which the outer diameter is constant along the entire length thereof or a tapered shape in which the outer diameter decreases from the proximal end side to the distal end side.

Examples of materials to be used for forming the coil body 20 include radiolucent materials such as stainless steel (e.g., SUS302, SUS304, and SUS316), an Ni-Ti alloy and a piano wire, and radiopaque materials such as platinum, gold, tungsten or alloys thereof. The coil body 20 may be formed entirely of the same material or materials that differ from one portion to another.

The coil body 20 is joined to the core wire 10. More specifically, the coil body 20 is joined to the core wire 10 through a distal joint part 31 formed near the distal end of the coil body 20, a proximal joint part 33 formed near the proximal end of the coil body 20, and an intermediate joint part 32 formed at a position between the distal end and the proximal end of the coil body 20. Examples of materials to be used for forming the distal joint part 31, the proximal joint part 33 and the intermediate joint part 32 include metal solder (e.g., Au-Sn alloy, Sn-Ag alloy, Sn-Pb alloy and Pb-Ag alloy), wax materials (e.g., aluminum alloy solder, silver solder and gold solder), and adhesives (e.g., epoxy-based adhesive). Materials to be used for forming the distal joint part 31, the proximal joint part 33 and the intermediate joint part 32 may be the same or differ from each other. The distal joint part 31, the proximal joint part 33 and the intermediate joint part 32 may each be formed entirely of the same material or formed of materials that differ from one portion to another.

### A-2. Detailed configuration of core wire 10

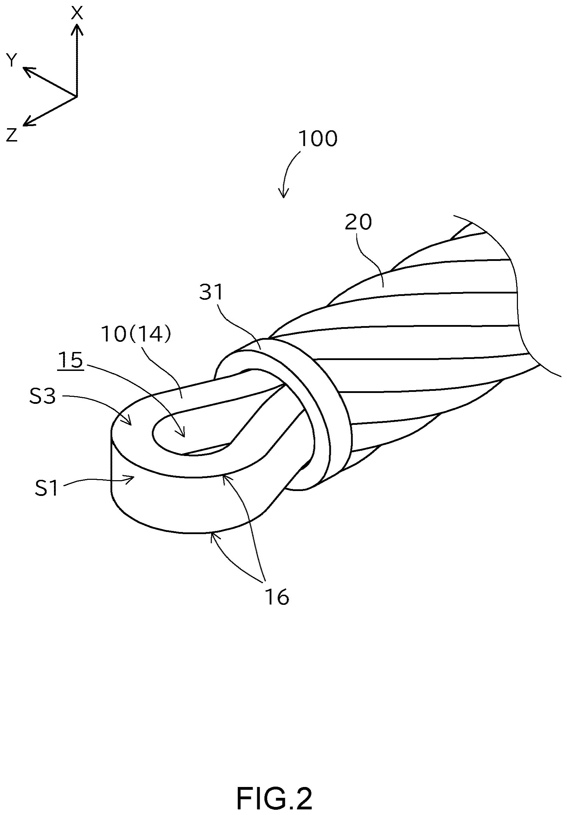

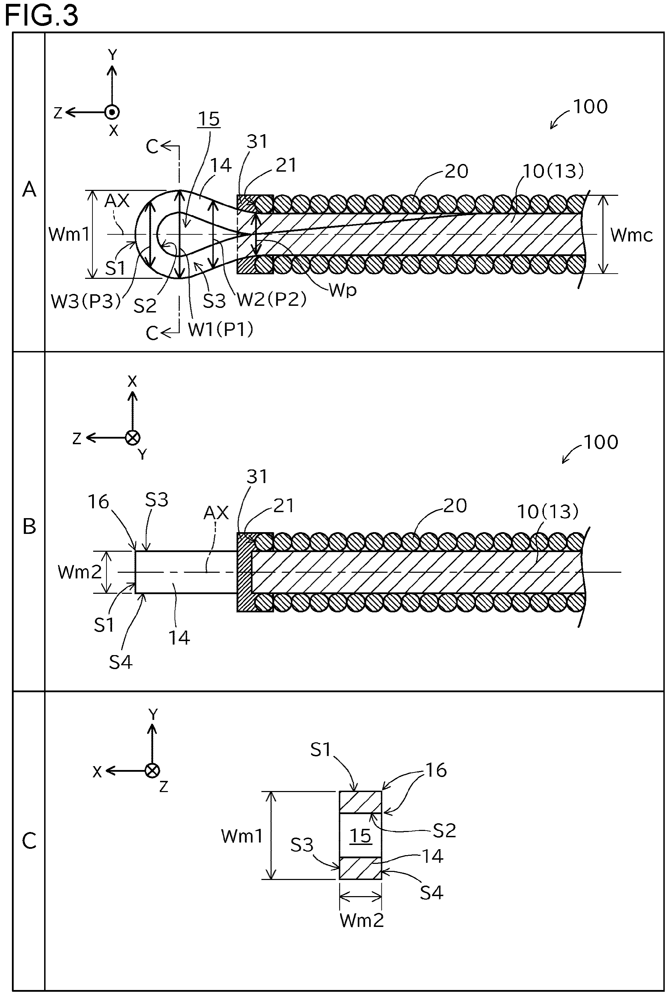

FIGS. 2 and 3 are diagrams that show the detailed configuration of the core wire 10 configuring the guide wire 100 in the first embodiment. FIG. 2 shows an enlarged view of the configuration of the distal end portion of the guide wire 100. Column A of FIG. 3 shows the configuration of one partial longitudinal section (YZ longitudinal section) of the distal end portion of the guide wire 100. Column B of FIG. 3 shows another partial longitudinal section (XZ longitudinal section) of the distal end portion of the guide wire 100. Column C of FIG. 3 shows the configuration of a transverse section (XY transverse section) of a protrusion 14 of the core wire 10 at the C-C position in the column A of FIG. 3. The protrusion 14 is not shown as a cross section in columns A and B of FIG. 3. The outer peripheral line of the protrusion 14 shown in column A of FIG. 3 denotes the contour of the protrusion 14 in the X-axis directional vision (specifically, the 1st directional vision). The outer peripheral line of the protrusion 14 shown in column B of FIG. 3 denotes the contour of the protrusion 14 in the Y-axis directional vision (specifically, the 2nd directional vision). Hereinafter, the contour in the X-axis directional vision is also referred to as a 1st contour. Hereinafter, the contour in the Y-axis directional vision is also referred to as a 2nd contour.

As shown in FIGS. 1 to 3, the protrusion 14 of the core wire 10 is located on the distal end side of the thin diameter part 13 covered by the coil body 20 and is a portion that protrudes to the farther distal end side beyond the distal end 21 of the coil body 20. The length of the protrusion 14 along the central axis AX ranges from about 0.5 mm to 2.0 mm, for example. The proximal end portion of the protrusion 14 is covered by the distal joint part 31 The remaining portion of the protrusion 14 is exposed externally.

The protrusion 14 of the core wire 10 has a loop part surrounding a through-hole 15 extending in the X-axis direction. More specifically, the protrusion 14 has a configuration in which a wire of a substantially rectangular cross section (an intermediate portion 43m of a metal wire 10m described later) is bent in the form of loop around the X axis and the loop is closed at the position of the proximal end. Therefore, as shown in the column A of FIG. 3, the 1st contour of the protrusion 14 has a shape in which the outer peripheral line has a curved portion. In the embodiment, the substantially entire shape of the outer peripheral line of the 1st contour of the protrusion 14 is substantially arcuate. In the 1st contour of the protrusion 14, an inner peripheral line that defines the through-hole 15 also has a curved portion. In the embodiment, the substantially entire shape of the inner peripheral line of the 1st contour of the protrusion 14 is substantially arcuate.

As shown in column A of FIG. 3, in the 1st contour of the protrusion 14, a width W1 at a 1st central axis position P1 in the vicinity of the substantial center of the protrusion 14 along the central axis AX is larger than a width W2 at a 2nd central axis position P2 closer to the proximal end side than the 1st central axis position P1. In the 1st contour of the protrusion 14, the width W1 at the 1st central axis position P1 is larger than a width W3 at a 3rd central axis position P3 closer to the distal end side than the 1st central axis position P1. More specifically, the 1st contour of the protrusion 14 has a width Wp that is substantially the same as that of a thin diameter part 13 of the core wire 10 at the position of the proximal end, and has a shape such that the width gradually increases from the proximal end to the distal end side, reaches its maximum value at the 1st central axis position P1, and gradually decreases from the 1st central axis position P1 to the distal end. The 1st central axis position P1 is located on the distal end side of the center along the central axis AX of the protrusion 14. In the embodiment, the maximum value of the width (=width W1 at the 1st central axis position P1) of the 1st contour of the protrusion 14 is the same as the maximum width Wm1 of the entire protrusion 14. The maximum width Wm1 of the protrusion 14 ranges from about 0.2 mm to 1.0 mm, for example.

In the following descriptions, the surface visually confirmed when the protrusion 14 is viewed from the X-axis positive direction is referred to as an upper surface S3 for convenience' sake, and the surface visually confirmed when the protrusion 14 is viewed from the X-axis negative direction is referred to as a lower surface S4 for convenience' sake. In the embodiment, the upper surface S3 and lower surface S4 of the protrusion 14 are substantially planar, and the upper surface S3 and the lower surface S4 are substantially in parallel to each other. Consequently, the width (can also be expressed as the thickness of the protrusion 14) of the 2nd contour of the protrusion 14 (see column B of FIG. 3) is a substantially constant value Wm2.

As shown in column A and column B of FIG. 3, the maximum value W1 of the width (=the maximum width Wm1 of the entire protrusion 14) of the 1st contour of the protrusion 14 is larger than the maximum value Wm2 of the width of the 2nd contour of the protrusion 14. That is, the protrusion 14 is flat-shape such that the width varies depending on the positions around the central axis AX. The maximum value W1 of the width (=Wm1) of the 1st contour of the protrusion 14 is preferably 1.2 times or more, more preferably 1.5 times or more, and even more preferably 1.8 times or more greater than the maximum value Wm2 of the width of the 2nd contour of the protrusion 14.

As shown in FIG. 2 and FIG. 3, the surface of the protrusion 14 has edges 16. Here, the edges 16 are each a boundary between two surfaces (ridge line). In the embodiment, the surface of the protrusion 14 has an edge 16 that is the boundary between an outer peripheral surface S1 (the surface configuring the outer peripheral line in the 1st contour) and an upper surface S3, an edge 16 that is the boundary between the outer peripheral surface S1 and a lower surface S4, an edge 16 that is the boundary between an inner peripheral surface S2 (the surface configuring an inner peripheral line in the 1st contour) and the upper surface S3, and an edge 16 that is the boundary between the inner peripheral surface S2 and the lower surface S4. Among these edges 16, the edge 16 that is the boundary between the outer peripheral surface S1 and the upper surface S3, and, the edge 16 that is the boundary between the outer peripheral surface S1 and the lower surface S4 are located on the 1st contour in the X-axis directional vision. The X-axis direction is an example of the 1st direction in claims. The Y-axis direction is an example of the 2nd direction in claims.

### A-3. Method for producing guide wire 100:

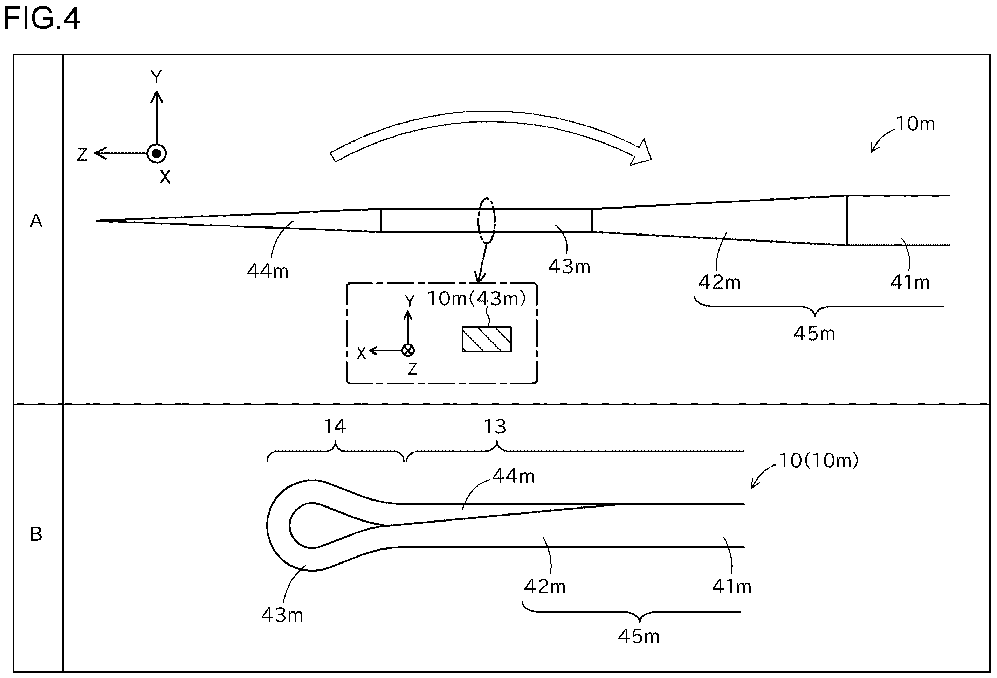

A guide wire 100 of this embodiment can be produced by the following method, for example. The first thing to do is to produce the core wire 10. FIG. 4 is a diagram showing an example of a method for producing the core wire 10 in the first embodiment. First, as shown in column A of FIG. 4, a metal wire 10m that is a material for forming the core wire 10 is produced. Column A of FIG. 4 shows a portion on the distal end side of the metal wire 10m, more specifically, a portion of the thin diameter part 13 of the core wire 10 and a portion serving as the protrusion 14. As shown in column A of FIG. 4, such one portion on the distal end side of the metal wire 10m has a 1st proximal end portion 41m, a 2nd proximal end portion 42m, an intermediate portion 43m, and a distal end portion 44m. Hereinafter, the 1st proximal end portion 41m and the 2nd proximal end portion 42m are together referred to as a proximal end portion 45m. The 1st proximal end portion 41m is a rod-shaped portion having a substantially constant diameter. The 2nd proximal end portion 42m is a portion extending from the distal end of the 1st proximal end portion 41m to the farther distal end direction and having a tapered shape in which the diameter gradually decreasing to the distal end. That is, the 2nd proximal end portion 42m has a transverse section having an area smaller than that of a transverse section of the 1st proximal end portion 41m. The intermediate portion 43m is a portion extending from the distal end of the 2nd proximal end portion 42m to the distal end direction and is rod-shaped to have a substantially constant diameter (the diameter substantially the same as that of the distal end of the 2nd proximal end portion 42m). As shown in column A of FIG. 4, the transverse section of the intermediate portion 43m is substantially rectangular. The distal end portion 44m is a portion extending from the distal end of the intermediate portion 43m to the distal end of the metal wire 10m and having a tapered shape in which the diameter gradually decreases to the distal end. The tapered shape of the 2nd proximal end portion 42m and the tapered shape of the distal end portion 44m are set so as to be consistent with each other, and the length of the 2nd proximal end part 42m and the length of the distal end part 44m are substantially the same. The metal wire 10m having such shape can be produced by preparing a wire with a substantially constant cross-sectional shape, followed by polishing of the wire, for example. The 1st proximal end portion 41m is an example of the 1st portion in claims and the 2nd proximal end portion 42m is an example of the 2nd portion in claims.

Next, as shown in columns A and B of FIG. 4, bending is performed to fold back the metal wire 10m, thereby forming a state in which the tapered surface of the distal end portion 44m is brought into contact with the tapered surface of the 2nd proximal end portion 42m, and the intermediate portion 43m is loop-shaped. Accordingly, the intermediate portion 43m becomes a loop part of the protrusion 14 of the core wire 10, and the distal end portion 44m and the 2nd proximal end portion 42m that are in contact with each other become rod-shaped to have substantially constant outer diameters, thereby configuring the thin diameter part 13 of the core wire 10, together with the 1st proximal end portion 41m. Portions serving as the tapered portion 12 and the thick diameter part 11 of the core wire 10 are formed in a portion located closer to the proximal end side than the 1st proximal end portion 41m in the metal wire 10m (omitted in FIGs). Therefore, the core wire 10 having the protrusion 14, the thin diameter part 13, the tapered portion 12, and the thick diameter part 11 is obtained by the above production method.

The thus produced core wire 10 is inserted to a hollow part of a separately prepared coil body 20. At this time, a state is created such that the protrusion 14 of the core wire 10 protrudes to the farther distal end side beyond the distal end 21 of the coil body 20. With this state, a distal joint part 31, an intermediate joint part 32 and a proximal joint part 33 for joining the coil body 20 and the core wire 10 are formed. The distal end portion 44m and the 2nd proximal end portion 42m in the metal wire 10m configuring the core wire 10 are joined with each other by the distal joint part 31 at a position covered by the coil body 20. For example, the guide wire 100 having the above configuration can be produced by the above-described method.

### A-4. Method for using guide wire 100:

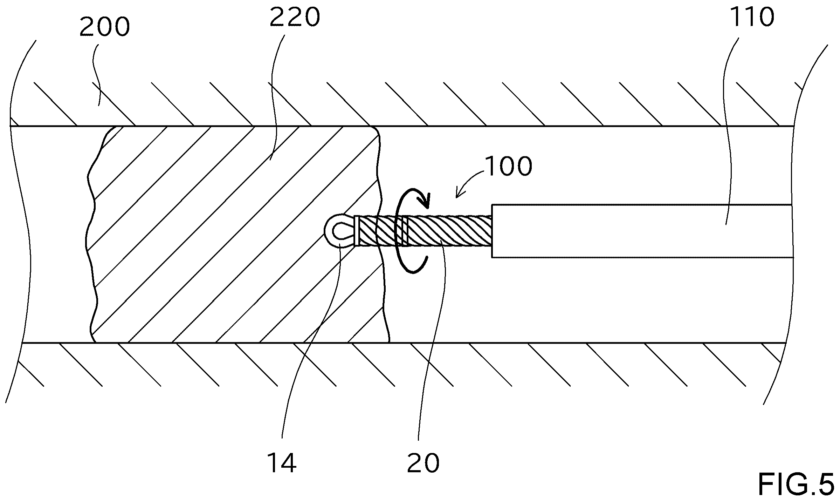

FIG. 5 is a diagram that shows an example of a method for using the guide wire 100 in the first embodiment. First, a professional such as a doctor inserts a guiding catheter 110 into a blood vessel 200 and then advances the guiding catheter 110 until the tip thereof reaches the position of a lesion 220 (for example, chronic total occlusion). Next, the professional inserts the guide wire 100 into the hollow part of the guiding catheter 110, and then advances the guide wire 100 to the lesion 220 within the blood vessel 200. When the guide wire 100 is advanced so that the distal end portion of the guide wire 100 protrudes from the distal end of the guiding catheter 110, the professional advances the guide wire 100 to further distal side while rotating the guide wire 100 around the central axis AX (clockwise rotation in this embodiment). The professional advances the guide wire 100 while rotating the guide wire 100 around the central axis AX as described above. Hence, if the protrusion 14 of the core wire 10 located at the distal end of the guide wire 100 comes into contact with the inner wall of the blood vessel 200, damage to the inner wall of the blood vessel 200 can be prevented.

Even after the distal end of the guide wire 100 reaches the lesion 220, the professional advances the guide wire 100 to the distal side while rotating it around the central axis AX. Accordingly, the protrusion 14 of the core wire 10 enters the lesion 220 and then the protrusion 14 is rotated around the central axis AX within the lesion 220, thereby cutting up the lesion 220. The coil body 20 having a spiral outer peripheral surface is also advanced to enter the lesion 220, and then the coil body 20 is rotated around the central axis AX within the lesion 220 and thus is screwed into the distal side within the lesion 220. As a result, the distal end portion of the guide wire 100 is surely advanced to the distal side in the lesion 220, thereby finally penetrating the lesion 220. Thereafter, the guide wire 100 having penetrated the lesion 220 is used as a rail to advance a catheter (not shown in the figure) to the position of the lesion 220.

When the guide wire 100 is withdrawn, the guide wire 100 is pulled back while rotating it around the central axis AX in the direction opposite to that upon insertion (counterclockwise rotation in this embodiment). Accordingly, rotation of the coil body 20 having the spiral outer peripheral surface enables smooth pulling back of the guide wire 100 and prevents damage to the inner wall of the blood vessel 200 even if the protrusion 14 of the core wire 10 located at the distal end of the guide wire 100 comes into contact with the inner wall of the blood vessel 200.

### A-5. Effect of first embodiment:

As described above, the guide wire 100 of the first embodiment includes the long main body 102 and the protrusion 14 protruding to the farther distal end of the main body 102. In the protrusion 14, the maximum value Wm1 of the width of the 1st contour when viewed from the X-axis direction orthogonal to the central axis AX of the main body 102 is larger than the maximum value Wm2 of the width of the 2nd contour when viewed from the Y-axis direction orthogonal to the central axis AX. In the X-axis directional vision, the 1st contour has a curved portion, and the width W1 of the 1st contour at the 1st central axis position P1 is larger than the width W2 of the 1st contour at the 2nd central axis position P2 located on the proximal end side of the 1st central axis position P1.

As described above, in the guide wire 100 of the embodiment, the protrusion 14 has a shape in which the 1st contour has a curved portion in the X-axis directional vision. Moreover, in the protrusion 14, the maximum value of the width of the 1st contour in the X-axis directional vision is larger than the maximum value of the width of the 2nd contour in the Y-axis directional vision. Specifically, the protrusion 14 has a non-rotationally symmetric shape. Accordingly, with the protrusion 14 located in the lesion 220, the guide wire 100 is rotated around the central axis AX to rotate the protrusion 14 around the central axis AX, so that the lesion 220 can be efficiently perforated by the curved portion of the contour of the protrusion 14. As described above, according to the guide wire 100 of the embodiment, the penetration performance can be improved, and thus the therapeutic efficiency of the guide wire 100 can be improved.

In the guide wire 100 of the embodiment, the X-axis direction that is a line-of-sight direction corresponding to the 1st contour is orthogonal to the Y-axis direction that is a line-of-sight direction corresponding to the 2nd contour. That is, the protrusion 14 is flat-shaped. Therefore, according to the guide wire 100 of the embodiment, resistance that occurs when the protrusion 14 is manipulated to enter the lesion 220 and when the protrusion 14 is advanced to the distal side in the lesion 220 can be reduced. As a result, the penetration performance of the guide wire 100 can be effectively improved.

In the guide wire 100 of the embodiment, the maximum value W1 of the width of the 1st contour of the protrusion 14 is the same as the maximum width Wm1 of the protrusion 14. That is, the direction in which 1st contour of the protrusion 14 has a curved portion is consistent with the direction in which the protrusion 14 has the maximum width Wm1. Therefore, according to the guide wire 100 of the embodiment, the width of a portion corresponding to the curved portion in the protrusion 14 is maximized, so as to be able to effectively improve the performance of perforating the lesion 220 by the rotation of the protrusion 14. As a result, the penetration performance of the guide wire 100 can be even more effectively improved.

In the guide wire 100 of the embodiment, the surface of the protrusion 14 has an edge16 that is a boundary of two surfaces. According to the guide wire 100 of the embodiment, the protrusion 14 is rotated around the central axis AX, enabling perforation of the lesion 220 in such a manner that the lesion 220 is cut up by the edge 16 on the surface of the protrusion 14. As a result, the penetration performance of the guide wire 100 can be even more effectively improved. In the guide wire 100 of the embodiment, the surface of the protrusion 14 has the edges 16 on the 1st contour in the X-directional vision . According to the guide wire 100 of the embodiment, the edge 16 can be arranged at the outermost peripheral positions on the rotational track when the protrusion 14 is rotated around the central axis AX, so that the edge16 of the rotating protrusion 14 can be surely brought into contact with the lesion 220, and the performance of perforating the lesion 220 by the rotation of the protrusion 14 can be effectively improved. As a result, the penetration performance of the guide wire 100 can be extremely effectively improved.

In the guide wire 100 of the embodiment, in the X-axis directional vision, the width W1 of the 1st contour at the 1st central axis position P1 of the protrusion 14 is larger than the width W3 of the 1st contour at the 3rd central axis position P3 located on the distal end side of the 1st central axis position P1. According to the guide wire 100 of the embodiment, the protrusion 14 has a portion the diameter of which increases and then decreases from the proximal end side to the distal end side, so that the portion on the distal end side of the protrusion 14 can be shaped to be highly capable of entering the lesion 220, as well as the width of a portion between the proximal end and the distal end of the protrusion 14 is relatively increased so as to be able to improve the performance of perforating the lesion 220 by the rotation of the protrusion 14. As a result, the penetration performance of the guide wire 100 can be further effectively improved.

In the guide wire 100 of the embodiment, the main body 102 includes a core wire 10 configured of a metal wire and a coil body 20 having a configuration in which one or more wires are wound around the outer periphery of the core wire 10, and being joined to the core wire 10. According to the guide wire 100 of the embodiment, the main body 102 includes the core wire 10 and the coil body 20, so that when the guide wire 100 is rotated around the central axis AX, the coil body 20 is also rotated around the central axis AX. Thus, the screw-in action of the coil body 20 having a spiral outer peripheral surface ensures the advancement of the protrusion 14 to the distal side in the lesion 220, and the penetration performance of the guide wire 100 can be even more effectively improved. According to the guide wire 100 of the embodiment, because of the presence of the coil body 20, the torquability of the distal end portion of the guide wire 100 can be improved, as well as the flexibility of the distal end portion of the guide wire 100 can be improved. Furthermore, in the unlikely event of the failure of the protrusion 14, the protrusion 14 can be prevented from remaining in the body cavity.

In the guide wire 100 of the embodiment, the protrusion 14 has a loop part. According to the guide wire 100 of the embodiment, the protrusion 14 can be shaped to have a portion with a relatively large width and a relatively thin linear portion, so that the lesion 220 can be effectively perforated by the rotating protrusion 14 and the penetration performance of the guide wire 100 can be extremely effectively improved.

In the guide wire 100 of the embodiment, the protrusion 14 has the loop part, and a metal wire 10m configuring the core wire 10 has a distal end portion 44m, a proximal end portion 45m, and an intermediate portion 43m located between the distal end portion 44m and the proximal end portion 45m. The distal end portion 44m and the proximal end portion 45m of the metal wire 10m are joined with each other at a position covered by the coil body 20. The intermediate portion 43m of the metal wire 10m configures a loop part of the protrusion 14 of the core wire 10. Therefore, according to the guide wire 100 of the embodiment, the core wire 10 having the loop-shaped protrusion 14 can be formed using a single metal wire 10m. As a result, compared to a configuration in which the protrusion 14 is formed as a separate body, the protrusion 14 can be prevented from leaving.

In the guide wire 100 of the embodiment, the proximal end portion 45m of the metal wire 10m has a 1st proximal end portion 41m and a 2nd proximal end portion 42m. The 2nd proximal end portion 42m is a portion adjacent to the distal end side with respect to the 1st proximal end portion 41m. The 2nd proximal end portion 42m has a transverse section with an area smaller than that of the transverse section of the 1st proximal end portion 41m and is joined to the distal end portion 44m of the metal wire 10m. Therefore, the guide wire 100 of the embodiment can prevent a situation in the core wire 10 such that the width of a portion (a portion on the distal end side of the thin diameter part 13) configured by joining the 2nd proximal end portion 42m and the distal end portion 44m of the metal wire 10m is excessively increased, resulting in an excessively increased rigidity gap between this portion and a portion configured of the 1st proximal end portion 41m (the remaining portion in the thin diameter part 13). Therefore, according to the guide wire 100 of the embodiment, the rigidity gap of the core wire 10 can be reduced and the durability and the manoeuvrability of the guide wire 100 can be improved.

In the guide wire 100 of the embodiment, the 2nd proximal end portion 42m of the metal wire 10m configuring the core wire 10 has a tapered shape in which the width gradually decreases from the boundary position thereof with the 1st proximal end portion 41m to the distal end side. Therefore, according to the guide wire 100 of the embodiment, compared to a configuration in which a step is made at the boundary position between the 2nd proximal end portion 42m and the 1st proximal end portion 41m, the rigidity gap of the metal wire 10 itself configuring the core wire 10 can be prevented from excessively increasing, and the durability and the manoeuvrability of the guide wire 100 can be effectively improved.

In the guide wire 100 of the embodiment, the coil body 20 is configured of a multi-thread coil formed by winding a plurality of wires around the outer periphery of the core wire 10. A multi-thread coil is better than a single-thread coil in terms of torquability and flexibility. Therefore, according to the guide wire 100 of the embodiment, the torquability and the flexibility of the distal end portion of the guide wire 100 can be effectively improved.

### B. Second embodiment

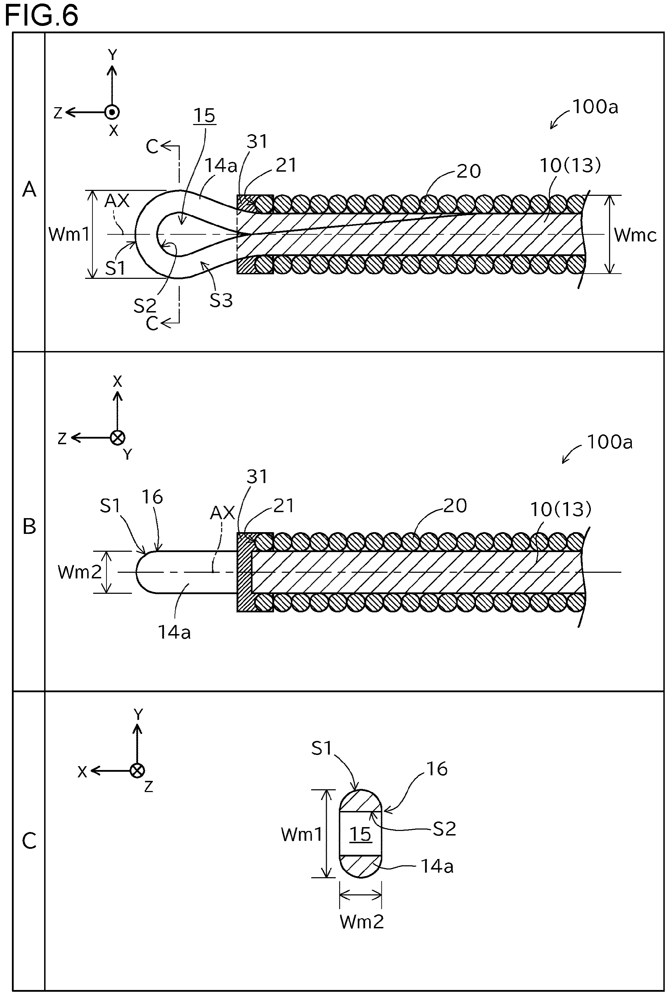

Fig. 6 is a diagram that shows the detailed configuration of the core wire 10 configuring a guide wire 100a of the second embodiment. Column A of FIG. 6 shows the configuration of one partial longitudinal section (YZ longitudinal section) of the distal end portion of the guide wire 100a. Column B of FIG. 6 shows the configuration of another partial longitudinal section (XZ longitudinal section) of the distal end portion of the guide wire 100a. Column C of FIG. 6 shows the configuration of a transverse section (XY transverse section) of a protrusion 14a of the core wire 10 at the C-C position in the column A of FIG. 6. The protrusion 14a is not shown as a cross section in columns A and B of FIG. 6. The outer peripheral line of the protrusion 14a shown in column A of FIG. 6 denotes the 1st contour of the protrusion 14a in the X-axis directional vision (specifically, the 1st directional vision). The outer peripheral line of the protrusion 14a shown in column B of FIG. 6 denotes the 2nd contour of the protrusion 14a in the Y-axis directional vision (specifically, the 2nd directional vision). Hereinafter, any component that is the same as that of the guide wire 100 of the above-described first embodiment, in the components of the guide wire 100a of the second embodiment, is omitted as appropriate by marking it with the same symbol.

The core wire 10 configuring the guide wire 100a of the second embodiment differs from that of the first embodiment in terms of the shape of the protrusion 14a. Specifically, the protrusion 14a in the second embodiment has a configuration wherein a wire of not a substantially rectangular cross section, but a substantially semicircular cross section (an intermediate portion 43m of a metal wire 10m described later) is bent in the form of loop around the X axis and the loop is closed at the position of the proximal end. In the second embodiment, as shown in columns B and C of FIG. 6, the linear portion of the substantially semicircular cross section of the above wire configuring the protrusion 14a faces a through-hole 15 and an arcuate portion in the substantially semicircular cross section faces the outer peripheral side. The other components of the protrusion 14a in the second embodiment are similar to those in the first embodiment.

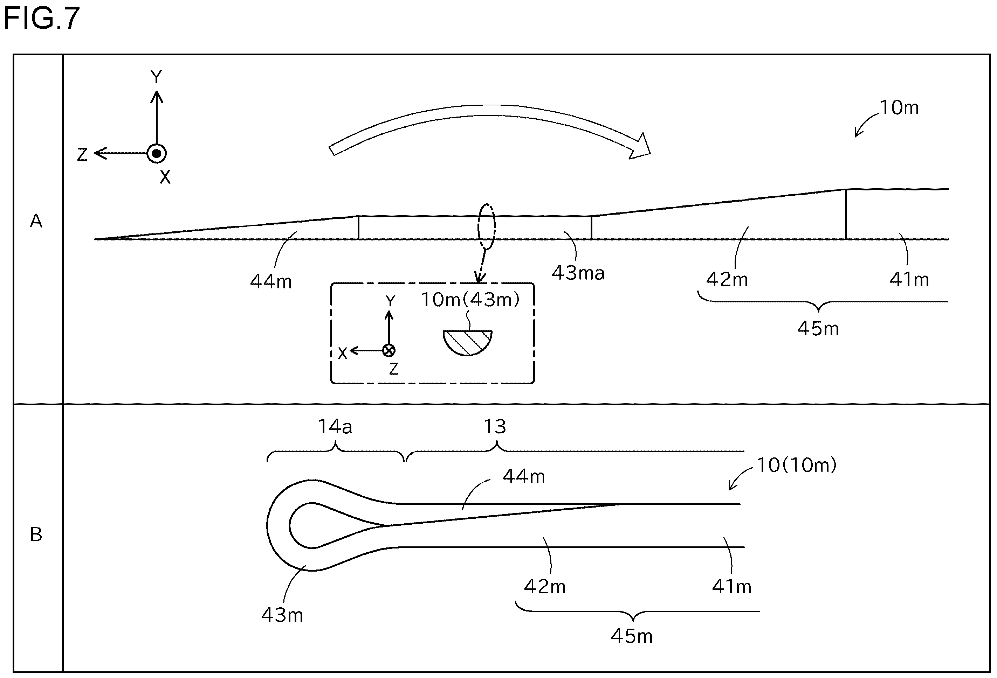

FIG. 7 is a diagram that shows an example of a method for producing the core wire 10 configuring the guide wire 100a of the second embodiment. When the core wire 10 in the second embodiment is produced, a metal wire 10m having a 1st proximal end portion 41m, 2nd proximal end portion 42m, an intermediate portion 43m, and a distal end portion 44m is produced in the same manner as in the first embodiment. At this time, as shown in column A of FIG. 7, the transverse section of the intermediate portion 43m is not formed into a substantially rectangular shape, but a substantially semicircular shape. As shown in columns A and B of FIG. 7, bending is performed to fold back the metal wire 10m, thereby causing the tapered surface of the distal end portion 44m to come into contact with the tapered surface of the 2nd proximal end portion 42m, and forming the loop-shaped intermediate portion 43m. Therefore, the intermediate portion 43m serves as the loop part of the protrusion 14a of the core wire 10.

As described above, the guide wire 100a of the second embodiment has the configuration similar to that of the guide wire 100 of the first embodiment, and thus it exerts effects (e.g., improvement in penetration performance) similar to those exerted by the guide wire 100 of the first embodiment described above.

### C. Third embodiment

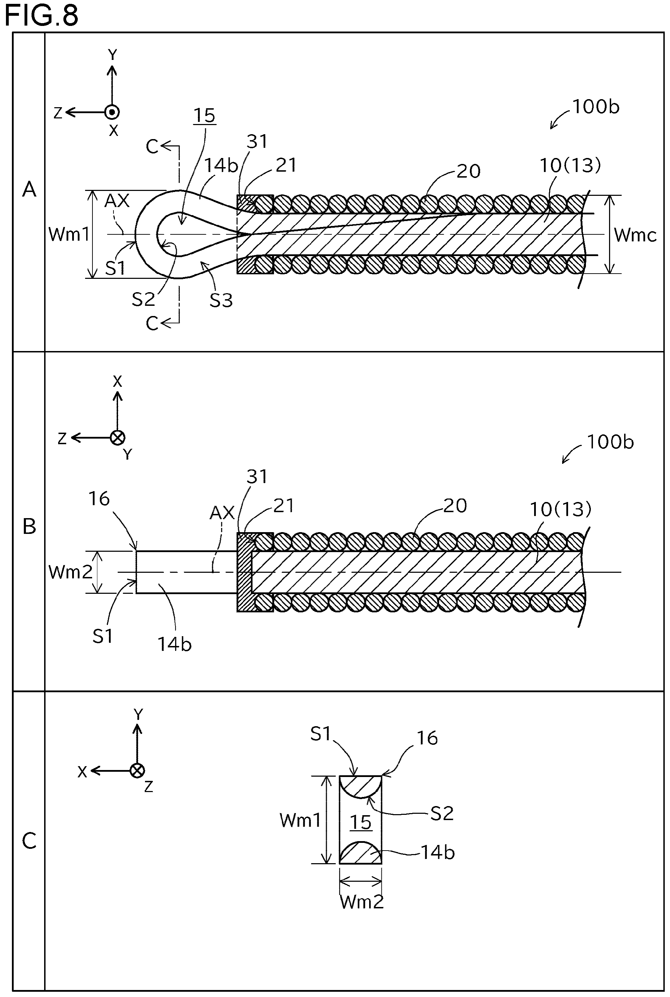

FIG. 8 is a diagram that shows the detailed configuration of the core wire 10 configuring a guide wire 100b of the third embodiment. Column A of FIG. 8 shows the configuration of one partial longitudinal section (YZ longitudinal section) of the distal end portion of the guide wire 100b. Column B of FIG. 8 shows the configuration of another partial longitudinal section (XZ longitudinal section) of the distal end portion of the guide wire 100b. Column C of FIG. 8 shows the configuration of a transverse section (XY transverse section) of the protrusion 14b of the core wire 10 at the C-C position in the column A of FIG. 8. The protrusion 14b is not shown as a cross section in columns A and B of FIG. 8. The outer peripheral line of the protrusion 14b shown in column A of FIG. 8 denotes the 1st contour of the protrusion 14b in the X-axis directional vision (specifically, the 1st directional vision). The outer peripheral line of the protrusion 14b shown in column B of FIG. 8 denotes the 2nd contour of the protrusion 14b in the Y-axis directional vision (specifically, the 2nd directional vision). Hereinafter, any component that is the same as that of the guide wire 100a of the above-described second embodiment, in the components of the guide wire 100b of the third embodiment, is omitted as appropriate by marking it with the same symbol.

The core wire 10 configuring the guide wire 100b of the third embodiment differs from that of the second embodiment in terms of the shape of the protrusion 14b. Specifically, the protrusion 14b in the third embodiment has a configuration similar to that of the protrusion14a in the second embodiment, such that the wire of the substantially semicircular cross section is bent into the form of loop around the X axis. As shown in columns B and C of FIG. 8, in the protrusion 14b, an arcuate portion in the substantially semicircular cross section of the above wire configuring the protrusion 14b faces the through-hole 15, and a linear portion of the substantially semicircular cross section faces the outer peripheral side. The other components of the protrusion 14b in the third embodiment are similar to those in the second embodiment.

When the core wire 10 in the third embodiment is produced, a metal wire 10m having a 1st proximal end portion 41m, a 2nd proximal end portion 42m, an intermediate portion 43m, and a distal end part 44m is produced in the same manner as in the second embodiment (see FIG. 7). At this time, the transverse section of the intermediate portion 43m is not formed into a substantially rectangular shape, but a substantially semicircular shape. Note that in the third embodiment, when bending is performed to fold back the distal end of the metal wire 10m, the folding-back direction is opposite to that of the second embodiment and the linear portion in the substantially semicircular cross section of the portion (intermediate portion 43m) configuring the protrusion 14b in the metal wire 10m is designed to face the outer peripheral side.

As described above, the guide wire 100b of the third embodiment has the configuration similar to that of the guide wire 100a of the second embodiment, and thus it exerts effects (e.g., improvement in penetration performance) similar to those exerted by the guide wire 100a of the second embodiment described above. In the guide wire 100b of the third embodiment, the surface of the protrusion14b has an edge 16 on the 1st contour in the X-directional vision. According to the guide wire 100b of the third embodiment, the edge 16 can be arranged at the outermost peripheral position on the rotational track when the protrusion 14b is rotated around the central axis AX, so that the edge 16 of the rotating protrusion 14b can be surely brought into contact with the lesion 220, and the performance of perforating the lesion 220 by the rotation of the protrusion 14b can be effectively improved. As a result, the penetration performance of the guide wire 100b can be extremely effectively improved.

### D. Fourth embodiment

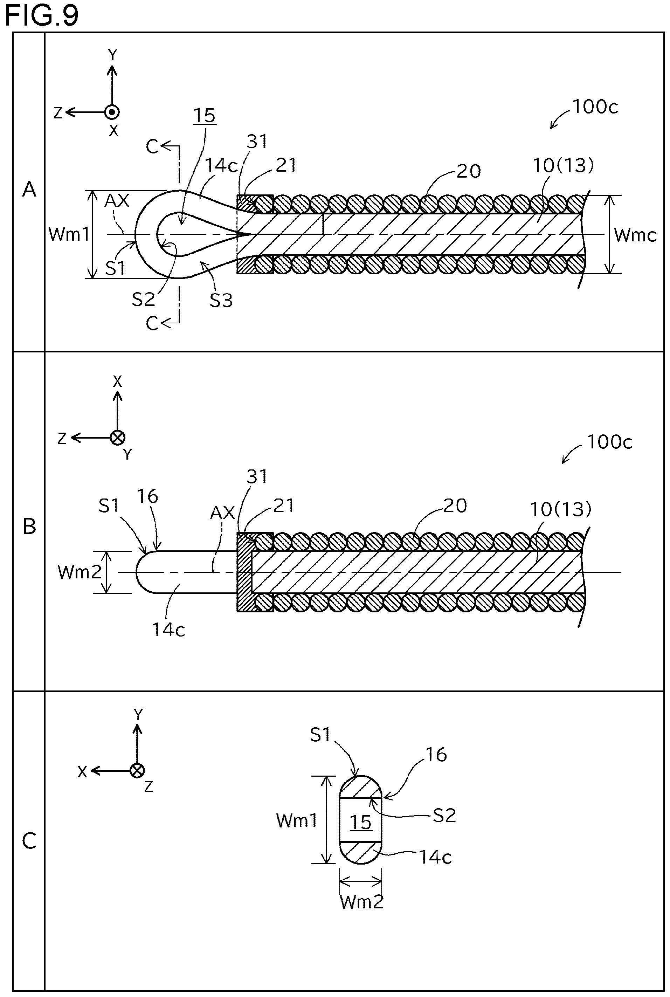

Fig. 9 is a diagram that shows the detailed configuration of the core wire 10 configuring a guide wire 100c of the fourth embodiment. Column A of FIG. 9 shows the configuration of one partial longitudinal section (YZ longitudinal section) of the distal end portion of the guide wire 100c. Column B of FIG. 9 shows the configuration of another partial longitudinal section (XZ longitudinal section) of the distal end portion of the guide wire 100c. Column C of FIG. 9 shows the configuration of a transverse section (XY transverse section) of a protrusion 14c of the core wire 10 at the C-C position in the column A of FIG. 9. The protrusion 14c is not shown as a cross section in columns A and B of FIG. 9. The outer peripheral line of the protrusion 14c shown in column A of FIG. 9 denotes the 1st contour of the protrusion 14c in the X-axis directional vision (specifically, the 1st directional vision). The outer peripheral line of the protrusion 14c shown in column B of FIG. 9 denotes the 2nd contour of the protrusion 14c in the Y-axis directional vision (specifically, the 2nd directional vision). Hereinafter, any component that is the same as that of the guide wire 100a of the above-described second embodiment, in the components of the guide wire 100c of the fourth embodiment, is omitted as appropriate by marking it with the same symbol.

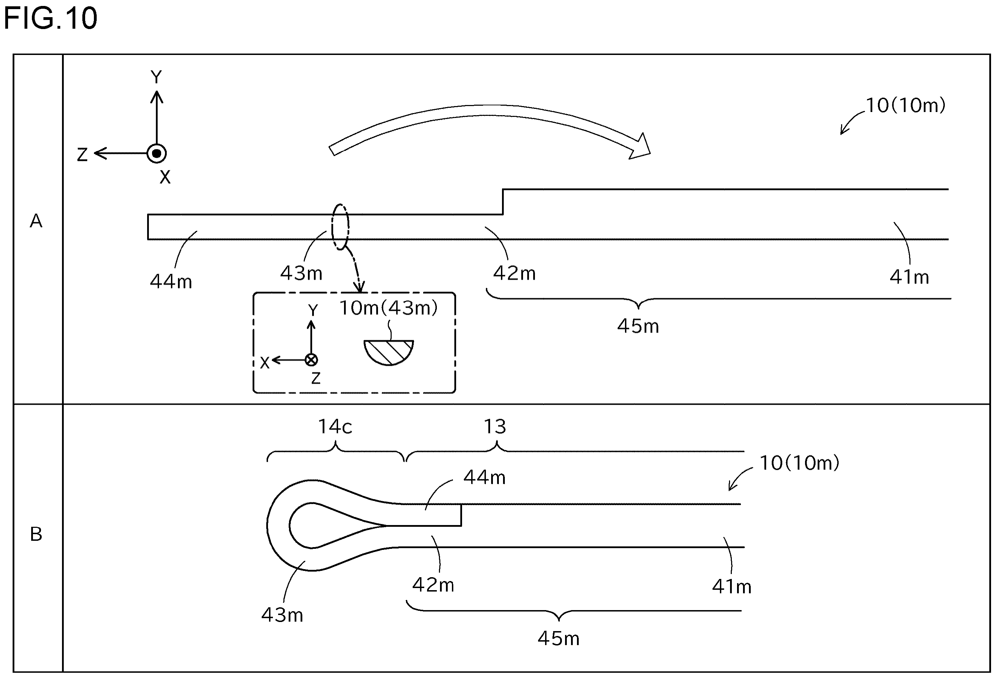

The guide wire 100c of the fourth embodiment differs from that of the second embodiment in terms of the configuration of the metal wire 10m configuring the core wire 10. FIG. 10 is a diagram that shows an example of a method for producing the core wire 10 configuring the guide wire 100c of the fourth embodiment. When the core wire 10 in the fourth embodiment is produced, a metal wire 10m having a 1st proximal end portion 41m, a 2nd proximal end portion 42m, an intermediate portion 43m, and a distal end portion 44m is produced in the same manner as in the second embodiment. At this time, as shown in column A of FIG. 10, a step is made at the boundary position between the 1st proximal end portion 41m and the 2nd proximal end portion 42m, resulting in the area of the transverse section of the 2nd proximal end portion 42m smaller than that of the transverse section of the 1st proximal end portion 41m. The 2nd proximal end portion 42m is a rod-shaped portion having a substantially constant diameter and the shape of the transverse section thereof is substantially the same as that of the transverse section of the intermediate portion 43m. As shown in columns A and B of FIG. 10, bending is performed to fold back the metal wire 10m with such configuration, thereby creating a state in which the outer peripheral surface of the distal end portion 44m comes into contact with the outer peripheral surface of the 2nd proximal end portion 42m, and the intermediate portion 43m is loop-shaped. Accordingly, the intermediate portion 43m is the loop part of the protrusion 14c of the core wire 10, the distal end portion 44m and the 2nd proximal end portion 42m are in contact with each other to form a rod having a substantially constant outer diameter, and thus the rod and the 1st proximal end portion 41m configure the thin diameter part 13 of the core wire 10.

As described above, the guide wire 100c of the fourth embodiment has the configuration similar to that of the guide wire 100a of the second embodiment, and thus it exerts effects (e.g., improvement in penetration performance) similar to those exerted by the guide wire 100a of the second embodiment described above.

### E. Fifth embodiment

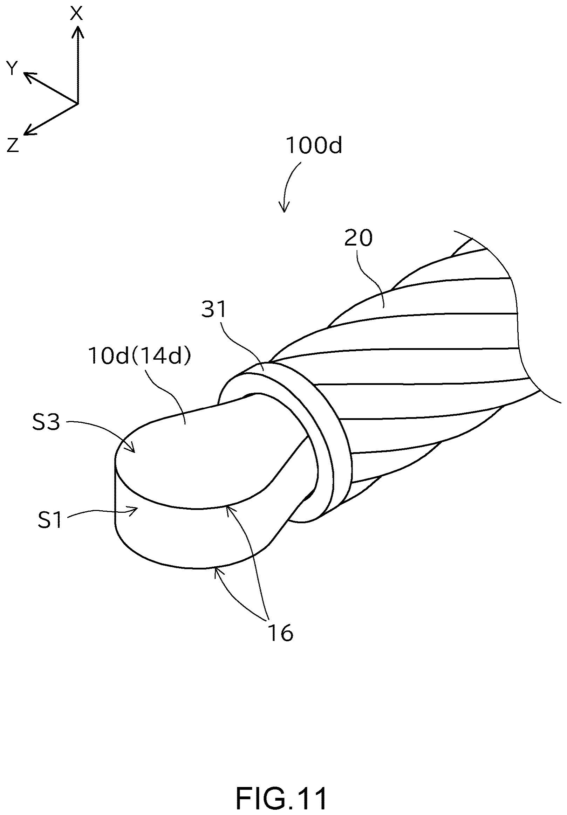

FIG. 11 is a diagram that shows the detailed configuration of the core wire 10 configuring a guide wire 100d in the fifth embodiment. FIG. 11 shows an enlarged view of the configuration of the distal end portion of the guide wire 100d. Hereinafter, any component that is the same as that of the guide wire 100 of the above-described first embodiment, in the components of the guide wire 100d of the fifth embodiment, is omitted as appropriate by marking it with the same symbol.

The core wire 10 configuring the guide wire 100d of the fifth embodiment differs from that of the first embodiment in terms of the shape of a protrusion 14d. Specifically, the protrusion 14d in the fifth embodiment does not have a shape having a loop part, but has a paddle shape. That is, the protrusion 14d in the fifth embodiment has a configuration in which a through-hole 15 is embedded in the protrusion 14 of the first embodiment. Accordingly, the YZ longitudinal section and the XZ longitudinal section of the protrusion14d in the fifth embodiment have similar shapes of those of the YZ longitudinal section and the XZ longitudinal section of the protrusion 14 in the first embodiment except the absence of the through-hole 15. Note that the protrusion 14d with such a shape can be produced by press working and polishing to wires, for example.

As described above, the guide wire 100d of the fifth embodiment has the configuration similar to that of the guide wire 100 of the first embodiment, and thus it exerts effects (e.g., improvement in penetration performance) similar to those exerted by the guide wire 100 of the first embodiment described above.

### F. Sixth embodiment

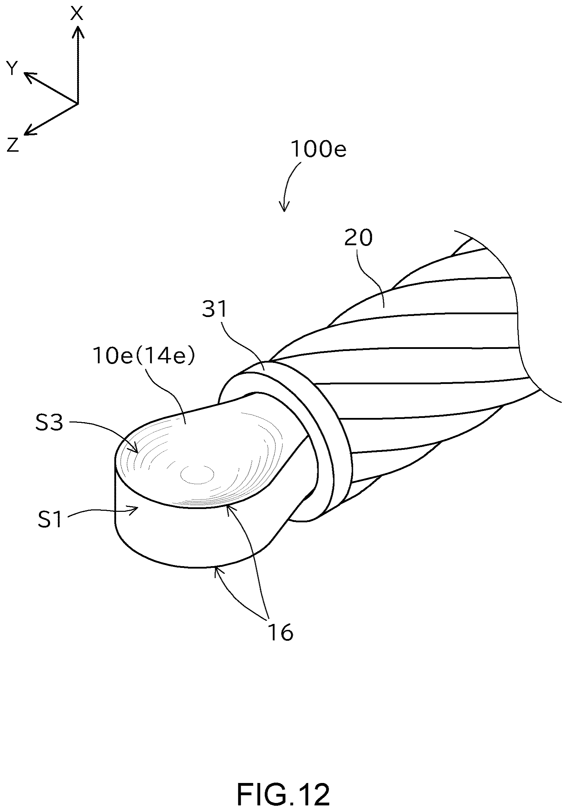

FIG. 12 is a diagram that shows the detailed configuration of the core wire 10 configuring a guide wire 100e in the sixth embodiment. FIG. 12 shows an enlarged view of the configuration of the distal end portion of a guide wire 100e. Hereinafter, any component that is the same as that of the guide wire 100 of the above-described first embodiment, in the components of the guide wire 100e of the sixth embodiment, is omitted as appropriate by marking it with the same symbol.

The core wire 10 configuring the guide wire 100e of the sixth embodiment differs from that of the first embodiment in terms of the shape of a protrusion 14e. Specifically, the protrusion 14e in the sixth embodiment does not have a shape having a loop part, but has a spoon shape. That is, the protrusion 14e in the sixth embodiment has a configuration wherein a through-hole 15 is embedded in the protrusion 14 in the first embodiment, and a recess is formed on an upper surface S3. Accordingly, the YZ longitudinal section and the XZ longitudinal section of the protrusion14e in the sixth embodiment have similar shapes of those of the YZ longitudinal section and the XZ longitudinal section of the protrusion 14 in the first embodiment except the presence of the recess instead of the through-hole 15. Note that the protrusion 14e having such a shape can be produced by press working and polishing to wires, for example.

As described above, the guide wire 100e of the sixth embodiment has the configuration similar to that of the guide wire 100 of the first embodiment, and thus it exerts effects (e.g., improvement in penetration performance) similar to those exerted by the guide wire 100 of the first embodiment described above.

### G. Modification example

The technology disclosed herein is not limited to the above-described embodiments, and can be modified in various forms without departing from the gist thereof. For example, the following modification examples are also possible.

The configurations of the guide wire 100 in the above embodiments are only examples and can be modified in various forms. For example, in the above embodiments, the coil body 20 is configured of a multi-thread coil formed by winding a plurality of wires. Each wire configuring the coil body 20 is a twisted wire. Each wire configuring the coil body 20 may not be a twisted wire but a single strand. The coil body 20 may be a single-thread coil formed by winding a single wire, instead of a multi-thread coil. The coil body 20 may be close-coiled or open-coiled.

In the above embodiments, the core wire 10 has the thick diameter part 11, the tapered portion 12, and the thin diameter part 13. The core wire 10 may not have at least one of these three parts, or may have other parts in addition to the three parts.

In the above embodiments, the shapes and the sizes of the protrusion 14 are only examples and may be other shapes and sizes, as long as in the 1st directional vision, the 1st contour of the protrusion 14 has a curved portion, and the width of the 1st contour at the 1st central axis position is larger than the width of the 1st contour at the 2nd central axis position located on the proximal end side of the 1st central axis position.. In the core wire 10, the protrusion 14 may be prepared as another body and then, may be brazed to the thin diameter part 13.

In the above embodiments, at least one of the distal joint part 31, the intermediate joint part 32 and the proximal joint part 33 may be omitted.

In the above embodiments, at least a portion of the guide wire 100 may be coated with a resin, for example.

The materials for each member in the above embodiments are only examples, and may be modified variously. The methods for producing the guide wire 100 in the above embodiments are only examples and can be modified variously.

The above embodiments are described using the guide wire 100 for treating an intravascular lesion. The technology disclosed herein is also similarly applicable to a medical device for treatment in a body cavity (e.g., blood vessel, gastrointestinal tract, and ureter).

### Reference Signs List

10: core wire, 10m: metal wire, 11: thick diameter part, 12: tapered portion, 13: thin diameter part, 14: protrusion, 15: through-hole, 16: edge, 20: coil body, 21: distal end, 31: distal joint part, 32: intermediate joint part, 33: proximal joint part, 41m: 1st proximal end portion, 42m: 2nd proximal end portion, 43m: intermediate portion 44m: distal end portion, 45m: proximal end portion, 100: guide wire, 102: main body, 110: guiding catheter, 200: blood vessel, 220: lesion, AX: central axis, S1: outer peripheral surface, S2: inner peripheral surface, S3: upper surface, S4: lower surface

## Claims

1. A medical device, comprising:
a long main body; and
a protrusion that protrudes to the farther distal end side beyond the distal end of the main body, in which the maximum value of the width of a contour of the protrusion when viewed from a 1st direction orthogonal to the central axis of the main body is larger than the maximum value of the width of a contour of the protrusion when viewed from a 2nd direction orthogonal to the central axis, wherein
in the 1st directional vision, the contour includes a curved portion, and the width of the contour at a 1st central axis position is larger than the width of the contour at a 2nd central axis position located on the proximal end side of the 1st central axis position.

2. The medical device according to claim 1,
wherein the 1st direction is orthogonal to the 2nd direction.

3. The medical device according to claim 2,
wherein the maximum value of the width of the contour in the 1st directional vision is the same as the maximum width of the protrusion.

4. The medical device according to any one of claims 1 to 3,
wherein the surface of the protrusion has an edge that is a boundary of two surfaces.

5. The medical device according to claim 4,
wherein the surface of the protrusion has the edge on the contour in the 1st directional vision.

6. The medical device according to any one of claims 1 to 5,
wherein in the 1st directional vision, the width of the contour at the 1st central axis position of the protrusion is larger than the width of the contour at a 3rd central axis position located on the distal end side of the 1st central axis position.

7. The medical device according to any one of claims 1 to 6,
wherein the main body includes a core wire configured of a metal wire and a coil body having a configuration in which one or more wires are wound around the outer periphery of the core wire, and being joined to the core wire.

8. The medical device according to any one of claims 1 to 7,
wherein the protrusion has a loop part.

9. The medical device according to claim 7,
wherein the protrusion has a loop part,
the metal wire configuring the core wire has a distal end portion, a proximal end portion, and an intermediate portion located between the distal end portion and the proximal end portion,
the distal end portion and the proximal end portion of the metal wire are joined with each other at a position covered by the coil body, and
the intermediate portion of the metal wire configures the loop part.

10. The medical device according to claim 9,
wherein the proximal end portion of the metal wire configuring the core wire has
a 1st portion and
a 2nd portion that is adjacent to the distal end side with respect to the 1st portion, is joined to the distal end portion of the metal wire, and has a transverse section having an area smaller than that of the transverse section of the 1st portion.

11. The medical device according to claim 10,
wherein the 2nd portion of the proximal end portion of the metal wire configuring the core wire has a tapered shape in which the width gradually decreases from the boundary position between the 2nd portion and the 1st portion to the distal end side.

12. The medical device according to any one of claims 9 to 11,
wherein the coil body is configured of a multi-thread coil formed by winding a plurality of the wires around the outer periphery of the core wire.

13. The medical device according to any one of claims 1 to 12,
wherein the 1st central axis position is located on the distal end side of the center of the central axis position of the protrusion.
